(19) Europäisches Patentamt
European Patent Office
Office européen des brevets

(11) Veröffentlichungsnummer : **0 127 782**
**B1**

(12) # EUROPÄISCHE PATENTSCHRIFT

(45) Veröffentlichungstag der Patentschrift : **11.11.87**

(51) Int. Cl.⁴ : **C 07 C179/10**, C 07 C178/00

(21) Anmeldenummer : **84105013.1**

(22) Anmeldetag : **04.05.84**

(54) **Verfahren zur Herstellung von wasserunlöslichen Peroxycarbonsäuren.**

(30) Priorität : **07.06.83 DE 3320497**

(43) Veröffentlichungstag der Anmeldung : **12.12.84 Patentblatt 84/50**

(45) Bekanntmachung des Hinweises auf die Patenterteilung : **11.11.87 Patentblatt 87/46**

(84) Benannte Vertragsstaaten : **AT BE CH DE FR GB IT LI LU NL SE**

(56) Entgegenhaltungen :
**FR-A- 2 101 175**
**GB-A- 2 032 421**
***Die Akte enthält technische Angaben, die nach dem Eingang der Anmeldung eingereicht wurden und die nicht in dieser Patentschrift enthalten sind.***

(73) Patentinhaber : **Degussa Aktiengesellschaft**
**Weissfrauenstrasse 9**
**D-6000 Frankfurt am Main 1 (DE)**

**Henkel Kommanditgesellschaft auf Aktien**
**Postfach 1100 Henkelstrasse 67**
**D-4000 Düsseldorf-Holthausen (DE)**

(72) Erfinder : **Dankowski, Manfred, Dr.**
**Stifterstrasse 14**
**D-8757 Karlstein (DE)**

**EP 0 127 782 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Peroxycarbonsäuren werden nicht nur als Oxydationsmittel in der organischen Synthese eingesetzt, sondern auch bei der Wäsche und/oder Bleiche von Textilien, da ihre Wirkung schon unterhalb 80 °C auftritt, siehe z. B. EU-OS 0 037 146 und US-PS 4 370 251.

Da aber feste, wasserunlösliche Peroxycarbonsäuren in reinem oder hochkonzentriertem Zustand thermisch und mechanisch sensibel sind, hatte man sich bisher mit deren Phlegmatisierung bzw. Stabilisierung befaßt und dabei in erster Linie verschiedenartige Derivate der Phosphor-, Phosphorig- oder Phosphonsäure eingesetzt, siehe z. B. EU-OS 0 037 146, sowie diverse Salze verschiedener Mineralsäuren, siehe z. B. BE-PS 560 389 ; EU-OS 0 048 290.

Die DE-PS 2 038 318 lehrt ein Verfahren zur Herstellung von Peroxycarbonsäurelösungen oder Peroxycarbonsäuren durch Umsetzung von Carbonsäureanhydriden oder Carbonsäuren mit Wasserstoffperoxid, wobei die Umsetzung in einer im allgemeinen zuvor entwässerten 5 bis 50 gew.-%igen Lösung von Wasserstoffperoxid in verschiedenen phosphororganischen Verbindungen erfolgt und die gebildete Peroxycarbonsäure nach Entfernung gegebenenfalls noch vorhandenen bzw. gebildeten Wassers aus der erhaltenen Lösung isoliert werden muß. Dieses Verfahren ist durch die Verwendung der phosphororganischen Verbindungen als Lösungsmittel, die erforderlichen Maßnahmen zur Entwässerung und Isolierung der Peroxycarbonsäure aus organischer Phase recht aufwendig und eignet sich darüber hinaus im wesentlichen nur zur Herstellung niederer aliphatischer Peroxycarbonsäuren.

Die GB-A-2 032 421 richtet sich auf ein Verfahren zur Herstellung lagerstabiler phlegmatisierter aliphatischer und/oder aromatischer Peroxycarbonsäuren durch Umsetzung der Carbonsäuren mit Wasserstoffperoxid in Gegenwart von Schwefelsäure und Zugabe mindestens einer Verbindung, welche mit Schwefelsäure ein Sulfatsalz bildet, zur Reaktionsmischung. Es wurde nicht erkannt, daß insbesondere bei der Herstellung der wasserunlöslichen Peroxycarbonsäuren Schaumprobleme auftreten, welche die Handhabung und technische Anwendung des Verfahrens erschweren oder gar unmöglich machen.

Zwar wurden außerdem auch die Schwierigkeiten, die bei der Umsetzung einer wasserunlöslichen Carbonsäure und wäßrigem Wasserstoffperoxid in Gegenwart einer Säure, also in einer Suspension, auftreten, eingehend untersucht, aber die vorgeschlagenen Lösungswege waren technisch sehr aufwendig, siehe z. B. US-PS 4 172 086 ; EU-OS 0 045 290.

Bei der Herstellung der höheren wasserunlöslichen Peroxycarbonsäuren, etwa ab $C_6$, hatte sich als sehr störend herausgestellt, daß die Reaktionsmischung aus Wasserstoffperoxid, wasserunlöslicher Carbonsäure und Säure, mit steigendem Molekulargewicht der Carbonsäure eine stark ansteigende Schaumbildung aufwies, wodurch die Handhabung dieser Reaktionsmischung äußerst erschwert wurde.

Aufgabe der Erfindung ist es daher, die Herstellung höherer Peroxycarbonsäuren ab $C_6$ in technisch einfacher Weise durchzuführen.

Es wurde nun gefunden, daß sich diese Aufgabe bei der Herstellung wasserunlöslicher Peroxycarbonsäuren mit 6 bis 16 Kohlenstoffatomen, bevorzugt 9 bis 13 Kohlenstoffatomen, durch Umsetzung der entsprechenden aliphatischen Carbonsäure mit 6 bis 16 C-Atomen bzw. der entsprechenden aromatischen Carbonsäure mit 7 bis 9 C-Atomen mit Wasserstoffperoxid in einer Reaktionsmischung, enthaltend die Carbonsäure, Wasserstoffperoxid, Wasser, Schwefelsäure und ein Phosphanoxid der allgemeinen Formel

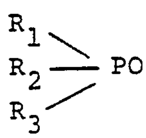

in der $R_1$, $R_2$, $R_3$ gleich oder verschieden sein können und einen Alkyl-, Cycloalkyl- oder Arylrest bedeuten, lösen läßt, wenn man das Phosphanoxid in Mengen von 0,01 bis 10 Gew.-%, bezogen auf eingesetzten Aktivsauerstoff, verwendet und Schwefelsäure und Carbonsäure im Molverhältnis von 1 bis 10 zu 1 einsetzt.

An und für sich können alle unter die oben beschriebene Formel fallenden Phosphanoxide für das erfindungsgemäße Verfahren eingesetzt werden, eine wesentliche Verbesserung des Herstellungsverfahrens wurde aber durch Trialkylphosphanoxide erreicht, deren einzelne Alkylgruppe 6 bis 10 Kohlenstoffatome enthält, wie z. B. : Tri-n-hexyl- ; Tri-n-heptyl- ; Tri-n-octyl- ; Tri-n-nonyl ; Tri-n-decyl-phosphanoxid.

Besonders bevorzugt ist Tri-n-octylphosphanoxid. Als Cycloalkylphosphanoxide kommen in Frage Tri-cyclopentyl- oder Tri-cyclohexylphosphanoxid.

Auch Triphenylphosphanoxid erwies sich als wirksam, ebenso Tribenzylphosphanoxid.

Die Phosphanoxide werden in Mengen von 0,01 bis 10 Gew.-%, bevorzugt 0,1 bis 7 Gew.-%, bezogen auf den eingesetzten Aktivsauerstoff, verwendet.

Man führt die Umsetzung mit einem Molverhältnis von Wasserstoffperoxid zu Carbonsäure von 1 bis 10 zu 1, bevorzugt 1,5 bis 3 zu 1, aus.

Von den obengenannten Mono- und Dicarbonsäuren sind besondern geeignet aliphatische Mono-und Dicarbonsäuren mit einer Gesamtzahl von 9 bis 13 C-Atomen, von den aromatischen Peroxycarbonsäuren die Phthalsäuren.

Besonders bevorzugt ist bei den aliphatischen Carbonsäuren Azelain-, Dodecandi- und Brassylsäure.

Bei der Durchführung des erfindungsgemässen Verfahrens erwies es sich ferner als günstig, Schwefelsäure und Carbonsäure im Molverhältnis 1 bis 10 zu 1, bevorzugt 2 bis 4 zu 1, einzusetzen.

Ferner zeigte sich, dass die besten Ergebnisse erhalten werden, wenn man das Phosphanoxid einer Mischung aus Wasserstoffperoxid und Schwefelsäure zusetzt und darauf die Carbonsäure in diese Mischung einführt.

Die Umsetzung wird bei Temperaturen zwischen 40-70 °C, bevorzugt bei 45-60 °C, durchgeführt.

Wasserstoffperoxid wird in Konzentrationen von 30 bis 99 Gew.-%, bevorzugt 40 bis 50 Gew.-%, eingesetzt ; Schwefelsäure in Konzentrationen von 20 bis 98 Gew.-%, bevorzugt 90 bis 98 Gew.-%.

Das Reaktionsprodukt wird üblicherweise durch Filtrieren oder Zentrifugieren aus der Reaktionsmischung abgetrennt und getrocknet. Ist das Produkt die reine Peroxycarbonsäure an sich, d. h. ohne die zusätzliche Phlegmatisierung mit Phlegmatisierungssalzen, so muss das Produkt mineralsäurefrei gewaschen werden.

Der technische Fortschritt des erfindungsgemässen Verfahrens liegt darin, dass durch die Verwendung eines Phosphanoxids die Viskosität der Reaktionsmischung aus Wasserstoffperoxid, Schwefelsäure, Wasser und Carbonsäure überraschenderweise stark herabgesetzt wird. So zeigte eine Reaktionsmischung ohne Zusatz von Phosphanoxid eine starke Schaumbildung, die sowohl eine Benetzung der Carbonsäure mit der Oxydationsmischung wie auch die Einstellung des Lösungsgleichgewichts im Sinne der Reaktion negativ beeinflusst. Dagegen wurde die Viskosität bei Zusatz von Trioctylphosphanoxid so stark herabgesetzt, dass eine technische Verwendung der Reaktionssuspension dadurch erst ermöglicht wird.

Die Mischung wurde besser rühr-, fließ- und pumpfähig. Auch liess sich der Restfeuchtegehalt, der aus den Peroxycarbonsäuren normalerweise schwer zu entfernen ist, erheblich vermindern.

Es ist nur bekannt, aus einer wissenschaftlichen Untersuchung, dass die Epoxidation mit Peroxysäuren durch Stoffe wie Dimethylformamid, Amin-, Phosphan- oder Arsanoxide gehemmt werden kann, s. Angew. Chemie 94 (1982), S. 750-766.

Daher war es völlig überraschend, dass die erfindungsgemässe Verwendung von Phosphanoxid in entscheidender Weise die Konsistenz der in Frage stehenden Reaktionsmischung beeinflusste.

Es wurde weiter gefunden, dass sich die entstehenden Peroxycarbonsäuren hervorragend stabilisieren ließen, wenn man vor, während oder nach der Umsetzung der Mischung ein zusätzliches Phlegmatisierungsmittel zusetzt.

Als solche Phlegmatisierungsmittel kommen Alkali-, Magnesium-, Erdalkali- oder Erdmetallsulfate oder Borsäure in Frage. Diese Stoffe können sowohl in fester Form oder als wässrige Lösungen bzw. Suspensionen zugesetzt werden. Es ist auch möglich, sie vor oder während der Umsetzung oder/und auf dem ausgefällten Produkt vor dessen Abtrennen aus der Reaktionslösung in situ zu erzeugen.

Besonders geeignet ist hierfür ein Verfahren, bei dem die Umsetzung der wasserunlöslichen Carbonsäure mit dem wässrigen Wasserstoffperoxid in Anwesenheit von Phosphanoxid bei 45-60 °C vorgenommen, darauf das Reaktionsgemisch auf Temperaturen von 45-5 °C, bevorzugt 40-32 °C, abgekühlt und eine wässrige Alkalisulfatlösung zugesetzt wird, der pH-Wert der Mischung auf 2-6 mit Hilfe von Alkalilaugen oder Alkalicarbonatlösungen eingestellt und danach das Reaktionsprodukt in bekannter Weise aufgearbeitet wird.

Als Alkalihydroxide oder Alkalicarbonate werden wässrige Lösungen mit 5-50 Gew.-%, bevorzugt 30-40 Gew.-% Alkalihydroxid, eingesetzt.

Von den drei Alkalihydroxiden bzw. -carbonaten ist das Natriumderivat besonders bevorzugt.

Der obengenannte pH-Wert von 2-6 kann ebenfalls eingestellt werden nach Beendigung der Reaktion zur Herstellung der Peroxycarbonsäure durch Zugabe von entsprechenden Mengen an Magnesiumhydroxid oder -oxid, oder von Erdalkalihydroxiden, wie deren Carbonaten, oder von Erdmetallhydroxiden, wie deren Carbonaten, ausserdem von Alkalialuminaten oder auch von Natriummetaborat. Auch diese Stoffe können in gelöster oder suspendierter Form, d. h. in wässrigem Medium, zugesetzt werden.

Das zusätzliche Phlegmatisierungsmittel soll bevorzugt in Mengen von 0 bis 80 Gew.-%, bezogen auf das fertige Produkt, anwesend sein.

Die Lagerstabilität wird durch die gleichzeitige Anwesenheit von Phosphanoxid und dem Phlegmatisierungsmittel in überraschend starker Weise erhöht. Wahrscheinlich liegt hier ein synergistischer Effekt der beiden Zusätze vor, siehe Beispiel 2.

Bei Verwendung von Natriumsulfat, das entweder fest oder in wässriger Lösung zugesetzt, oder in situ gebildet wird — auch können alle drei Möglichkeiten gleichzeitig verwendet werden — wird der Restfeuchtegehalt der Peroxycarbonsäuren nach dem Zentrifugieren um bis zu 50 % vermindert.

Besonders stark tritt dieser Effekt auf, wenn die in situ Bildung von Natriumsulfat aus der im Reaktionsgemisch vorhandenen Schwefelsäure und zugesetzter Natronlauge bei Temperaturen oberhalb des Umwandlungspunktes von Natriumsulfat-Dekahydrat zu wasserfreiem Natriumsulfat, d. h. bei Temperaturen ab 32,3-32,4 °C, vorgenommen wird.

Die Erfindung wird anhand der folgenden Beispiele erläutert. Beispiel 1 wird ohne zusätzlichen Stabilisator, allein in Gegenwart von Trioctylphosphanoxid durchgeführt ; die Beispiele 2 bis 5 in

Anwesenheit des zusätzlichen Stabilisators Natriumsulfat.

Hierbei bedeuten :

DPDA = Diperoxydodecandisäure

AO = Aktivsauerstoffgehalt

% = Gewichtsprozent.

Bei dem Versuch, Dodecandisäure mit 70 Gew.%igem Wasserstoffperoxid und 96 Gew.%iger Schwefelsäure in einem (beispielhaften) Molverhältnis von 1 : 3 : 4 umzusetzen, trat ein starker, beständiger Schaum — wahrscheinlich durch partielle Zersetzung von Wasserstoffperoxid — auf. Dieser Schaum ließ sich auch durch Rühren nicht abbauen, so daß eine einwandfreie Durchführung der gewünschten Umsetzung, vor allem bei einem Versuch in größerem Maßstab, nicht möglich war.

Wird dagegen gemäß den nachfolgenden Beispielen 1-9 verfahren, so trat nicht nur eine so unwesentliche Schaumbildung auf, daß problemlos in größerem Maßstab gearbeitet werden konnte, sondern die an die Umsetzung sich anschließende Neutralisation der restlichen Schwefelsäure ließ sich durch gute Verteilung des Neutralisierungsmittels ohne jede Gefahr der Zersetzung von Wasserstoffperoxid durch lokale Ansammlung von Alkali durchführen. Ferner war die schließlich erhaltene Suspension fließfähig und ohne weiteres zentrifugierbar.

Beispiel 1

In eine Oxidationsmischung, bestehend aus 255 g Wasserstoffperoxid (50 gew.-%ig), 510 g Schwefelsäure (96 gew.-%ig) und 64 g Natriumsulfatlösung (13 gew.-%ig), sowie 0,4 g Tri-n-octylphosphanoxid, werden 287,5 g Dodecandisäure eindosiert und 8 h unter Rühren auf 60 °C erhitzt. Nach dem Abkühlen auf 20 °C wird mit 1 l destilliertem Wasser versetzt und abgesaugt. Der Rückstand wird mit kaltem destilliertem Wasser gewaschen und getrocknet.

Die Ausbeute an Persäure beträgt : 295 g ≙ 90,1 % der Theorie

An Gesamt-AO-Gehalt wird gefunden : 11,56 %

Die Dodecandisäurebilanz beträgt : 96,7 %

Der Gehalt an DPDA beträgt : 94,3 %.

Beispiel 2

In eine Oxidationsmischung, bestehend aus 170 g Wasserstoffperoxid (50 gew.-%ig) und 204 g Schwefelsäure (96 gew.-%ig), sowie 1,3 g Tri-n-octylphosphanoxid, werden 115 g Dodecandisäure eindosiert und 8 h unter Rühren auf 50 °C erhitzt. Nach dem Abkühlen auf 8 °C wird der Ansatz bei dieser Temperatur mit 300 g Natriumsulfatlösung (13 gew.-%ig) versetzt und anschliessend mit 507 g Natriumhydroxidlösung (30 gew.-%ig) bis zum Erreichen des pH-Wertes von 3,5 neutralisiert. Anschliessend wird zentrifugiert und getrocknet. Trocknung bei 40 °C.

Die Ausbeute an Persäure beträgt : 103,5 g ≙ 79,0 % d. Th.

An Gesamt-AO-Gehalt wird gefunden : 3,86 %

Die Dodecandisäurebilanz beträgt : 88,5 %

Gehalt und Lagerstabilität :

| | 0 Wochen | 4 Wochen | 8 Wochen | 12 Wochen |
|---|---|---|---|---|
| DPDA | 30,9 | 30,5 | 30,5 | 30,1 |
| AO | 3,86 | 3,85 | 3,85 | 3,73 |

Beispiel 3

In eine Oxidationsmischung, bestehend aus 102 g Wasserstoffperoxid (50 gew.-%ig), 204 g Schwefelsäure (96 gew.-%ig) und 25 g Natriumsulfatlösung (12 gew.-%ig), sowie 0,16 g Tri-n-octylphosphanoxid, werden 115 g Dodecandisäure eindosiert und 8 h unter Rühren auf 60 °C erhitzt. Nach dem Abkühlen auf 40 °C wird der Ansatz bei dieser Temperatur mit 526 g Natriumsulfatlösung (30 gew.-%ig) versetzt und anschliessend mit 523 g Natriumhydroxidlösung (30 gew.-%ig) bis zum Erreichen des pH-Wertes von 3,5 neutralisiert und mit 160 g festem Natriumsulfat konditioniert. Anschliessend wird zentrifugiert und getrocknet. Trocknung bei 40 °C.

Die Ausbeute an Persäure beträgt : 110,4 g ≙ 84,3 % d. Th.

An Gesamt-AO-Gehalt wird gefunden : 4,25 %

Die Dodecandisäurebilanz beträgt : 91,8 %

Der Gehalt an DPDA beträgt : 34,3 %.

Beispiel 4

In eine Oxidationsmischung, bestehend aus 102 g Wasserstoffperoxid (50 gew.-%ig), 204 g

Schwefelsäure (96 gew.-%ig) und 25 g Natriumsulfatlösung (13 gew.-%ig), sowie 0,16 g Tri-n-octylphosphanoxid, werden 115 g Dodecandisäure eindosiert und 8 h unter Rühren auf 60 °C erhitzt. Nach dem Abkühlen auf 40 °C wird der Ansatz bei dieser Temperatur mit 394 g Natriumsulfatlösung (30 gew.-%ig) versetzt und anschliessend mit 523 g Natriumhydroxidlösung (30 gew.-%ig) bis zum Erreichen des pH-Wertes von 3,5 neutralisiert und mit 160 g festem Natriumsulfat konditioniert. Anschliessend wird zentrifugiert und getrocknet.

Die Ausbeute an Persäure beträgt : 112,8 g ≙ 86,1 % d. Th.
An Gesamt-AO-Gehalt wird gefunden : 4,27 %
Die Dodecandisäurebilanz beträgt ; 95,5 %
Der Gehalt an DPDA beträgt : 34,4 %.

Beispiel 5

In eine Oxidationsmischung, bestehend aus 127 g Wasserstoffperoxid (40 gew.-%ig) und 204 g Schwefelsäure (96 gew.-%ig), sowie 0,16 g Tri-n-octylphosphanoxid, werden 115 g Dodecandisäure eindosiert und 8 h unter Rühren auf 60 °C erhitzt. Nach dem Abkühlen auf 40 °C wird der Ansatz bei dieser Temperatur mit 394 g Natriumsulfatlösung (30 gew.-%ig) versetzt und anschliessend mit 521 g Natriumhydroxidlösung (30 gew.-%ig) bis zum Erreichen des pH-Wertes von 3,5 neutralisiert und mit 160 g festem Natriumsulfat konditioniert. Anschliessend wird zentrifugiert und getrocknet. Trocknung bei 40 °C.

Die Ausbeute an Persäure beträgt : 110,4 g ≙ 84,3 % d. Th.
An Gesamt-AO-Gehalt wird gefunden : 4,27 %
Die Dodecandisäurebilanz beträgt : 91,8 %
Der Gehalt an DPDA beträgt : 34,4 %.

Beispiel 6

In eine Oxidationsmischung, bestehend aus 102 g Wasserstoffperoxid (50 gew.-%ig), 204 g Schwefelsäure (96 gew.-%ig) und 25 g Natriumsulfatlösung (13 gew.-%ig) sowie 1,6 g Tri-n-octylphosphanoxid, werden 115 g Dodecandisäure eindosiert und 8 h unter Rühren auf 60 °C erhitzt. Nach dem Abkühlen auf 40 °C wird der Ansatz bei dieser Temperatur mit 263 g Natriumsulfatlösung (30 gew.-%ig) versetzt und anschliessend mit 526 g Natriumhydroxidlösung (30 gew.-%ig) bis zum Erreichen des pH-Wertes von 3,5 neutralisiert und mit 160 g festem Natriumsulfat konditioniert. Anschliessend wird zentrifugiert und getrocknet.

Die Ausbeute an Persäure beträgt : 118,3 g ≙ 90,3 % d. Th.
An Gesamt-AO-Gehalt wird gefunden : 4,25 %
Die Dodecandisäurebilanz beträgt : 97,5 %
Der Gehalt an DPDA beträgt : 34,4 %.

Beispiel 7

In eine Oxidationsmischung, bestehend aus 102 g Wasserstoffperoxid (50 gew.-%ig), 204 g Schwefelsäure (96 gew.-%ig) und 25 g Natriumsulfatlösung (13 gew.-%ig) sowie 0,16 g Tri-n-octylphosphanoxid, werden 115 g Dodecandisäure eindosiert und 6 h unter Rühren auf 60 °C erhitzt. Nach dem Abkühlen auf 40 °C wird der Ansatz bei dieser Temperatur mit 526 g Natriumsulfatlösung (30 gew.-%ig) versetzt und anschliessend mit 521 g Natriumhydroxidlösung (30 gew.-%ig) bis zum Erreichen des pH-Wertes von 3,5 neutralisiert und mit 120 g Natriumsulfat konditioniert. Anschliessend wird separiert und getrocknet.

Die Ausbeute an Persäure beträgt : 122,8 g ≙ 93,7 % d. Th.
An Gesamt-AO-Gehalt wird gefunden : 4,76 %
Die Dodecandisäurebilanz beträgt : 97,7 %
Der Gehalt an DPDA beträgt : 40,0 %.

Beispiel 8

In eine Oxidationsmischung, bestehend aus 102 g Wasserstoffperoxid (50 gew.-%ig), 204 g Schwefelsäure (96 gew.-%ig) und 25 g Natriumsulfatlösung (13 gew.-%ig) sowie 0,16 g Tri-n-octylphosphanoxid, werden 115 g Dodecandisäure eindosiert und 6 h unter Rühren auf 60 °C erhitzt. Nach dem Abkühlen auf 40 °C wird der Ansatz bei dieser Temperatur mit 526 g Natriumsulfatlösung (30 gew.-%ig) versetzt und anschliessend mit 521 g Natriumhydroxidlösung (30 gew.-%ig) bis zum Erreichen des pH-Wertes von 3,5 neutralisiert und mit 80 g Natriumsulfat konditioniert. Anschliessend wird separiert und getrocknet.

Die Ausbeute an Persäure beträgt : 118,1 g ≙ 90,1 % d. Th.
An Gesamt-AO-Gehalt wird gefunden : 5,30 %
Die Dodecandisäurebilanz beträgt : 96,5 %

Der Gehalt an DPDA beträgt : 43,4 %.

Beispiel 9

In eine Oxidationsmischung, bestehend aus 102 g Wasserstoffperoxid (50 gew.-%ig), 204 g Schwefelsäure (96 gew.-%ig) und 25 g Natriumsulfatlösung (13 gew.-%ig) sowie 0,16 g Tri-n-octylphosphanoxid, werden 115 g Dodecandisäure eindosiert und 6 h unter Rühren auf 60 °C erhitzt. Nach dem Abkühlen auf 40 °C wird der Ansatz bei dieser Temperatur mit 526 g Natriumsulfatlösung (30 gew.-%ig) versetzt und anschliessend mit 521 g Natriumhydroxidlösung (30 gew.-%ig) bis zum Erreichen des pH-Wertes von 3,5 neutralisiert und mit 40 g Natriumsulfat konditioniert. Anschliessend wird separiert und getrocknet.

Die Ausbeute an Persäure beträgt : 116,7 g ≙ 89,1 % d. Th.

An Gesamt-AO-Gehalt wird gefunden : 7,02 %

Die Dodecandisäurebilanz beträgt : 94,5 %

Der Gehalt an DPDA beträgt : 57,5 %.

**Patentansprüche**

1. Verfahren zur Herstellung von wasserunlöslichen Peroxycarbonsäuren mit 6 bis 16 C-Atomen, bevorzugt 9 bis 13 C-Atomen, durch Umsetzung der entsprechenden aliphatischen Carbonsäure mit 6 bis 16 C-Atomen bzw. aromatischen Carbonsäure mit 7 bis 9 C-Atomen mit Wasserstoffperoxid in einer Reaktionsmischung, enthaltend die Carbonsäure, Wasserstoffperoxid, Wasser, Schwefelsäure und ein Phosphanoxid der allgemeinen Formel

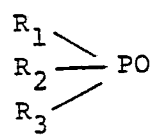

in der $R_1$, $R_2$ oder $R_3$ gleich oder verschieden sind und einen Alkyl-, Cycloalkyl- oder Arylrest bedeuten, dadurch gekennzeichnet, daß man das Phosphanoxid in Mengen von 0,01 bis 10 Gew.-%, bezogen auf den eingesetzten Aktivsauerstoff, verwendet und Schwefelsäure und Carbonsäure im Molverhältnis von 1 bis 10 zu 1 einsetzt.

2. Verfahren nach Anspruch 1, dadurch gekennzeichnet, dass man die Umsetzung in Gegenwart eines Trialkylphosphanoxides, dessen Alkylgruppe je 4 bis 18 C-Atome enthalten, durchführt.

3. Verfahren nach Anspruch 1 und 2, dadurch gekennzeichnet, dass man die Umsetzung in Gegenwart eines Trialkylphosphanoxides, dessen Alkylgruppe je 6 bis 10 C-Atome, bevorzugt 8 Kohlenstoffatome, wie Tri-n-octylphosphanoxid, enthalten, durchführt.

4. Verfahren nach Anspruch 1 bis 3, dadurch gekennzeichnet, daß man das Phosphanoxid in Mengen von 0,1 bis 7 Gew.-%, bezogen auf den eingesetzten Aktivsauerstoff, verwendet.

5. Verfahren nach Anspruch 1 bis 4, dadurch gekennzeichnet, dass man die Umsetzung mit einem Molverhältnis von Wasserstoffperoxid zu Carbonsäure von 1 bis 10 zu 1, bevorzugt 1,5 bis 3 zu 1, vornimmt.

6. Verfahren nach Anspruch 1 bis 5, dadurch gekennzeichnet, dass man das Phosphanoxid der Mischung aus Wasserstoffperoxid und Schwefelsäure zusetzt und dann die Umsetzung mit Carbonsäure durchführt.

7. Verfahren nach Anspruch 1 bis 6, dadurch gekennzeichnet, daß man Schwefelsäure und Carbonsäure im Molverhältnis von 2 bis 4 zu 1 einsetzt.

8. Verfahren nach Anspruch 1 bis 7, dadurch gekennzeichnet, dass man als zusätzliches Phlegmatisierungsmittel eine wässrige Alkali-, Magnesium-, Erdalkali- oder Erdmetallsulfatlösung, bevorzugt eine Natriumsulfatlösung, vor, während oder nach der Umsetzung zusetzt.

9. Verfahren nach Anspruch 1 bis 7, dadurch gekennzeichnet, dass man als zusätzliches Phlegmatisierungsmittel Alkalialuminat, bevorzugt Natriumaluminat, in wässriger Lösung vor, während oder nach der Umsetzung zusetzt.

10. Verfahren nach Anspruch 1 bis 7, dadurch gekennzeichnet, dass man als zusätzliches Phlegmatisierungsmittel eine wässrige Lösung von Natriummetaborat während oder nach der Umsetzung zugibt.

11. Verfahren nach Anspruch 1 bis 10, dadurch gekennzeichnet, dass man die Umsetzung zwischen Carbonsäure, Wasserstoffperoxid, Schwefelsäure, Wasser und Phosphanoxid bei Temperaturen von 40 bis 70 °C, bevorzugt 45 bis 60 °C, vornimmt und nach Abkühlung auf 40 bis 5 °C das zusätzliche Phlegmatisierungsmittel zufügt.

12. Verfahren nach Anspruch 1 bis 11, dadurch gekennzeichnet, dass man die Umsetzung von wasserunlöslicher Carbonsäure und wässrigem Wasserstoffperoxid in Gegenwart von Schwefelsäure und Phosphanoxid bei Temperaturen von 45 bis 60 °C vornimmt, darauf das Reaktionsgemisch auf

Temperaturen von 45-5 °C, bevorzugt auf 40-32 °C, abkühlt und eine wässrige Alkalisulfatlösung zusetzt, worauf man den pH-Wert der Reaktionsmischung auf 2 bis 6 mit Alkalilaugen oder Alkalicarbonatlösungen einstellt und das reaktionsprodukt in bekannter Weise aufarbeitet.

13. Verfahren nach Anspruch 1 bis 12, dadurch gekennzeichnet, dass man als Carbonsäure aliphatische Mono- oder Dicarbonsäure, bevorzugt Azelainsäure, Dodecandisäure oder Brassylsäure, einsetzt.

**Claims**

1. A process for the production of water-insoluble peroxycarboxylic acids containing from 6 to 16 carbon atoms, preferably from 9 to 13 carbon atoms, by reaction of the corresponding aliphatic carboxylic acid containing from 6 to 16 carbon atoms or aromatic carboxylic acid containing from 7 to 9 carbon atoms with hydrogen peroxide in a reaction mixture containing the carboxylic acid, hydrogen peroxide, water, sulphuric acid and a phosphane oxide corresponding to the general formula

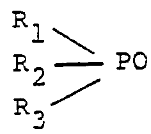

in which $R_1$, $R_2$ or $R_3$ are the same or different and represent an alkyl, cycloalkyl or aryl radical, characterised in that the phosphane oxide is used in quantities of from 0.01 to 10 % by weight, based on the active oxygen used, and sulphuric acid and carboxylic acid are used in a molar ratio of between 1 and 10 to 1.

2. A process according to Claim 1, characterised in that the reaction is carried out in the presence of a trialkyl phosphane oxide whose alkyl group contains from 4 to 18 carbon atoms in each case.

3. A process according to Claims 1 and 2, characterised in that the reaction is carried out in the presence of a trialkyl phosphane oxide whose alkyl group contains from 6 to 10 carbon atoms, preferably 8 carbon atoms each, such as tri-n-octyl phosphane oxide.

4. A process according to Claims 1 to 3, characterised in that the phosphane oxide is used in quantities of from 0.1 to 7 % by weight, based on the active oxygen used.

5. A process according to Claims 1 to 4, characterised in that the reaction is carried out with a molar ratio of hydrogen peroxide to carboxylic acid of between 1 and 10 to 1, preferably between 1.5 and 3 to 1.

6. A process according to Claims 1 to 5, characterised in that the phosphane oxide is added to the mixture of hydrogen peroxide and sulphuric acid and the reaction with carboxylic acid is then carried out.

7. A process according to Claims 1 to 6, characterised in that sulphuric acid and carboxylic acid are used in a molar ratio of between 2 and 4 to 1.

8. A process according to Claims 1 to 7, characterised in that an aqueous alkali, magnesium, alkaline earth or earth metal sulphate solution, preferably a sodium sulphate solution is added as additional desensitizing agent before, during or after the reaction.

9. A process according to Claims 1 to 7, characterised in that alkali aluminate, preferably sodium aluminate, in an aqueous solution is added as additional desensitizing agent before, during or after the reaction.

10. A process according to Claims 1 to 7, characterised in that an aqueous solution of sodium metaborate is added as additional desensitizing agent during or after the reaction.

11. A process according to Claims 1 to 10, characterised in that the reaction between carboxylic acid, hydrogen peroxide, sulphuric acid, water and phosphane oxide is carried out at temperatures of from 40 to 70 °C, preferably 45 to 60 °C and the additional desensitizing agent is added after cooling to between 40 and 5 °C.

12. A process according to Claims 1 to 11, characterised in that the reaction of water-insoluble carboxylic acid and aqueous hydrogen peroxide is carried out in the presence of sulphuric acid and phosphane oxide at temperatures of from 45 to 60 °C, the reaction mixture is then cooled to temperatures of from 45 to 5 °C, preferably from 40 to 32 °C and an aqueous alkali sulphate solution is added, whereupon the pH of the reaction mixture is adjusted to between 2 and 6 with alkali liquors or alkali carbonate solutions and the reaction product is worked up in known manner.

13. A process according to Claims 1 to 12, characterised in that aliphatic mono or dicarboxylic acid, preferably azelaic acid, dodecane diacid or brassic acid is used as carboxylic acid.

**Revendications**

1. Procédé pour la préparation d'acides peroxycarboxyliques insolubles dans l'eau, ayant de 6 à 16 atomes de carbone, de préférence de 9 à 13 atomes de carbone, par réaction des acides carboxyliques

aliphatiques appropriés, ayant de 6 à 16 atomes de carbone, ou des acides carboxyliques aromatiques appropriés ayant de 7 à 9 atomes de carbone, avec du peroxyde d'hydrogène dans un mélange réactionnel contenant l'acide carboxylique, du peroxyde d'hydrogène, de l'eau, de l'acide sulfurique et un phosphanoxyde de formule générale :

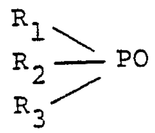

dans laquelle $R_1$, $R_2$, $R_3$ sont identiques ou différents et représentent un reste alkyle, cycloalkyle ou aryle, caractérisé en ce que l'on utilise le phosphanoxyde en quantités allant de 0,01 à 10 % en poids, par rapport à l'oxygène actif mis en réaction, et on utilise l'acide sulfurique et l'acide carboxylique en un rapport molaire allant de 1 à 10 : 1.

2. Procédé selon la revendication 1, caractérisé en ce que l'on effectue la réaction en présence d'un trialkylphosphanoxyde dont les groupes alkyle contiennent chacun de 4 à 18 atomes de carbone.

3. Procédé selon la revendication 1 ou 2, caractérisé en ce que l'on effectue la réaction en présence d'un trialkylphosphanoxyde dont les groupes alkyle contiennent chacun de 6 à 10 atomes de carbone, de préférence 8 atomes de carbone, tel que le tri-n-octylphosphanoxyde.

4. Procédé selon l'une quelconque des revendications 1 à 3, caractérisé en ce que l'on utilise le phosphanoxyde en quantités allant de 0,1 à 7 % en poids, par rapport à l'oxygène actif mis en réaction.

5. Procédé selon l'une quelconque des revendications 1 à 4, caractérisé en ce que l'on effectue la réaction avec un rapport molaire du peroxyde d'hydrogène à l'acide carboxylique allant de 1 à 10 : 1, de préférence de 1,5 à 3 : 1.

6. Procédé selon l'une quelconque des revendications 1 à 5, caractérisé en ce que l'on ajoute le phosphanoxyde au mélange composé de peroxyde d'hydrogène et d'acide sulfurique et on effectue ensuite la réaction avec l'acide carboxylique.

7. Procédé selon l'une quelconque des revendications 1 à 6, caractérisé en ce que l'on utilise l'acide sulfurique et l'acide carboxylique en un rapport molaire allant de 2 à 4 : 1.

8. Procédé selon l'une quelconque des revendications 1 à 7, caractérisé en ce que l'on ajoute en tant qu'agent stabilisant additionnel, une solution aqueuse d'un sulfate de métal alcalin, de magnésium, de métal alcalino-terreux ou de métal terreux, de préférence une solution de sulfate de sodium, avant, pendant ou après la réaction.

9. Procédé selon l'une quelconque des revendications 1 à 7, caractérisé en ce que l'on ajoute en tant qu'agent stabilisant additionnel, un aluminate de métal alcalin, de préférence l'aluminate de sodium, en solution aqueuse, avant, pendant ou après la réaction.

10. Procédé selon l'une quelconque des revendications 1 à 7, caractérisé en ce que l'on ajoute en tant qu'agent stabilisant additionnel, une solution aqueuse de métalborate de sodium, pendant ou après la réaction.

11. Procédé selon l'une quelconque des revendications 1 à 10, caractérisé en ce que l'on effectue la réaction entre l'acide carboxylique, le peroxyde d'hydrogène, l'acide sulfurique, l'eau et le phosphanoxyde, à des températures allant de 40 à 70 °C, de préférence de 45 à 60 °C, et après refroidissement à 40-5 °C, on ajoute l'agent stabilisant additionnel.

12. Procédé selon l'une quelconque des revendications 1 à 11, caractérisé en ce que l'on effectue la réaction de l'acide carboxylique insoluble dans l'eau et du peroxyde d'hydrogène en solution aqueuse, en présence d'acide sulfurique et d'un phosphanoxyde, à des températures de 45 à 60 °C, à la suite de quoi on refroidit le mélange réactionnel à des températures de 45-5 °C, de préférence à 40-32 °C, et on y ajoute une solution aqueuse d'un sulfate alcalin, puis on ajuste le pH du mélange réactionnel à 2-6 avec des solutions d'hydroxydes de métaux alcalins ou des solutions de carbonates de métaux alcalins, et on achève le traitement du produit de la réaction, de façon connue.

13. Procédé selon l'une quelconque des revendications 1 à 12, caractérisé en ce que l'on utilise en tant qu'acide carboxylique un acide mono- ou dicarboxylique, de préférence l'acide azélaïque, l'acide dodécanedioïque ou l'acide brassylique.